Europäisches Patentamt

European Patent Office

Office européen des brevets

(19)

(11) Publication number : **0 396 974 B1**

(12) **EUROPEAN PATENT SPECIFICATION**

(45) Date of publication of patent specification :
**11.08.93 Bulletin 93/32**

(51) Int. Cl.⁵ : **C07C 17/00, C07C 21/18**

(21) Application number : **90108005.1**

(22) Date of filing : **27.04.90**

(54) **Process for preparing fluoroalkyl vinyl compound.**

(30) Priority : **27.04.89 JP 109537/89**

(43) Date of publication of application :
**14.11.90 Bulletin 90/46**

(45) Publication of the grant of the patent :
**11.08.93 Bulletin 93/32**

(84) Designated Contracting States :
**DE FR GB IT**

(56) References cited :
**FR-A- 2 583 039**
**US-A- 2 697 124**
**US-A- 2 802 887**
**M. HUDLICKY: "REACTIONS IN ORGANIC CHEMISTRY", pages 63-67, 1984, Ellis Horwood Publications, Chichester, GB**

(73) Proprietor : **DAIKIN INDUSTRIES, LIMITED**
**Umeda Center Building 4-12 Nakazaki-nishi 2-chome Kita-ku**
**Osaka 530 (JP)**

(72) Inventor : **Yoshida, Tutomu**
**1-1-006, Higashiota**
**Ibaraki-shi, Osaka-fu (JP)**
Inventor : **Yamaguchi, Fumihiko**
**2-29-20-302 Tamagushi**
**Ibaraki-shi, Osaka-fu (JP)**
Inventor : **Nakagawa, Youichi**
**2-21-21, Hitotsuya**
**Settsu-shi, Osaka-fu (JP)**

(74) Representative : **Hansen, Bernd, Dr. Dipl.-Chem. et al**
**Hoffmann, Eitle & Partner Patent- und Rechtsanwälte, Postfach 81 04 20**
**W-8000 München 81 (DE)**

# Description

The present invention relates to a process for preparing a fluoroalkyl vinyl compound.

When a vinyl compound is prepared from a vinyl halide through reductive dehalogenation, there is used a metal reagent which can selectively proceed the desired reaction such as lithium hydride (cf. J. Am. Chem. Soc., 1973, 95, 6452-4), metal sodium (cf. J. Am. Chem. Soc., 1968, 90, 3595), and a Grignard reagent (cf. J. Orgmetal. Chem., 1976, 113, 107-113). However, these materials are not industrially attractive because of their high reactivity and dangerous properties and because of troublesome treatment of waste materials.

It is proposed to use a palladium catalyst in hydrogenation (cf. U.S. Patent No. 2,697,124). This U.S. Patent uses, as a starting material, a saturated fluorohalogenated carbon and does not intend to selectively prepare one product.

It is known that, when a vinyl halide is used as a starting material and a palladium catalyst is used, hydrogen atoms are added to a double bond selectively (cf. Izv. Akad. Nauk. SSSR, Ser. Khim., 1983, (12), 2775-81).

In view of the above prior arts, to suppress the addition reaction of hydrogen to the double bond and to increase the selectivity in the reductive dehalogenation, a gaseous catalytic reaction is proposed (cf. U.S. Patent No. 2,802,887 and Japanese Patent Publication No. 2324/1971). However, in these prior arts, a starting material is CClF=CF$_2$, and a product is limited to CHF=CF$_2$. To suppress side reactions such as addition of hydrogen to the double bond, reaction conditions such as contact time and temperature should be strictly controlled. In addition, even if the reaction conditions are well controlled, a yield of the desired product is low.

One object of the present invention is to provide a process for preparing a fluoroalkyl vinyl compound without using dangerous highly reactive reagents.

Another object of the present invention is to provide a process for preparing a fluoroalkyl vinyl compound which process requires no strict control of the reaction conditions.

Further object of the present invention is to provide a process for preparing a fluoroalkyl vinyl compound in a high yield.

These and other objects are achieved by a process for preparing a fluoroalkyl vinyl compound of the formula:

$$R_fCF = CH_2 \quad (I)$$

wherein $R_f$ is a fluoroalkyl group having 1 to 10 carbon atoms, which process comprises reacting a fluoroalkyl vinyl halide of the formula:

$$R_fCF = CHX \quad (II)$$

or

$$R_fCF = CX_2 \quad (III)$$

wherein $R_f$ is the same as defined above, and X is a chlorine atom, a bromine atom or an iodine atom with hydrogen in the presence of a palladium catalyst.

The fluoroalkyl group $R_f$ may be a straight one or a branched one. The number of the fluorine atoms in the fluoroalkyl group is preferably larger than, more preferably at least twice larger than the number of the carbon atoms in the group. Preferred examples of the fluoroalkyl group are CF$_3$-, CF$_3$C(CF$_2$)$_3$-, CF$_3$(CF$_2$)$_5$-, HCF$_2$-, H(CF$_2$)$_3$- or (CF$_3$)$_2$CFCF$_2$-.

Specific examples of the fluoroalkyl vinyl halides (II) are H(CF$_2$)$_3$CF=CHCl, H(CF$_2$)$_3$CF=CHI and CF$_3$CF=CHCl.

Specific examples of the fluoroalkyl vinyl halides (III) are H(CF$_2$)$_3$CF=CCl$_2$, H(CF$_2$)$_3$CF=CI$_2$ and CF$_3$CF=CCl$_2$.

The amount of hydrogen is usually from 0.1 to 10 moles, preferably from 0.5 to 1.5 moles per one mole of the fluoroalkyl vinyl halide.

The catalyst is palladium, which is usually supported on activated carbon. The amount of supported catalyst is from 0.01 to 0.1 part per one part of the fluoroalkyl vinyl halide when the supported amount of palladium is from 0.5 to 20 % by weight based on the weight of the activated carbon.

The reaction can be carried out in the presence or absence of a solvent. Preferably, the reaction is carried out in the solvent. Usually, water is used as the solvent, although an organic solvent such as an alcohol may be used. An amount of the solvent is 0.3 to 20 times the volume of the fluoroalkyl vinyl halide.

To remove a hydrogen halide which is generated during the reaction, a base such as sodium hydroxide, potassium hydroxide or an amine may be used.

The process of the present invention can be carried out continuously or preferably batchwise. In the batchwise process, hydrogen is supplied to the reaction system by blowing during the reaction or charging by pressurization before the start of the reaction. Hydrogen may be diluted with an inert gas such as nitrogen.

The reaction temperature is usually from 5 to 200°C, preferably from 10 to 150°C. The reaction pressure is usually from 50,7 kPa to 5066,2 kPa (0.5 to 50 atm), preferably from 101,3 kPa to 1013,2 kPa (1 to 10 atm). The reaction time is from 1 to 30 seconds in the continuous process, or 1 to 10 hours in the batchwise process.

The reactor may be made of any conventionally used material such as glass, iron, nickel or an alloy of iron or nickel.

The reaction product can be recovered from the reaction mixture by a per se conventional method. For example, after completion of the reaction the organic phase is washed with water to remove the residual acidic substances and then distilled to separate the product from the unreacted materials.

The present invention will be illustrated by the following Examples.

## Example 1

In a 50 ml flask equipped with a stirrer and a thermometer, $H(CF_2)_3CF=CHCl$ (4.61 g, 0.02 mole), 0.5 % palladium/carbon catalyst (0.3 g), potassium hydroxide (1.12 g, 0.02 mole) and water (5 ml) were charged. After purging the internal atmosphere with hydrogen, hydrogen gas kept at 101,3 kPa (1 atm.) was introduced in the flask at room temperature (25°C) for 6 hours. The reaction system absorbed 520 cc of hydrogen gas. After the reaction, the organic phase was analyzed with gas chromatography (Porapak Type Q) to find that the conversion was 85.0 % by mole and the selectivity of $H(CF_2)_3CF=CH_2$ was 95.9 % by mole. The by-product was $H(CF_2)_3CHFCH_3$.

## Example 2

In the same flask as used in Example 1, $H(CF_2)_3$-$CF=CHCl$ (4.61 g, 0.02 mole), 5 % palladium/carbon catalyst (0.3 g), potassium hydroxide (1.12 g, 0.02 mole) and water (5 ml) were charged. After purging the internal atmosphere with hydrogen, hydrogen gas kept at 101,3 kPa (1 atm) was introduced in the flask at room temperature (25°C) for 9 hours. The reaction system absorbed 480 cc of hydrogen gas. After the reaction, the organic phase was analyzed with gas chromatography (Porapak Type Q) to find that the conversion was 73.4 % by mole and the selectivity of $H(CF_2)_3CF=CH_2$ was 90.2 % by mole. The by-product was $H(CF_2)_3CHFCH_3$.

## Example 3

In the same flask as used in Example 1, a mixture of 45 % by mole of $H(CF_2)_3CF=CCl_2$ and 55 % by mole of $H(CF_2)_3CF=CHCl$ (4.92 g, 0.02 mole), 0.5 % palladium/carbon catalyst (0.3 g), potassium hydroxide (1.63 g, 0.03 mole) and water (5 ml) were charged. After purging the internal atmosphere with hydrogen, hydrogen gas kept at 101,3 kPa (1 atm) was introduced in the flask at room temperature (25°C) for 10 hours. The reaction system absorbed 780 cc of hydrogen gas. After the reaction, the organic phase was analyzed with gas chromatography (Porapak Type Q) to find that the conversion was 81.0 % by mole and the selectivity of $H(CF_2)_3CF=CH_2$ was 93.2 % by mole. The by-product was $H(CF_2)_3CHFCH_3$.

## Example 4

In the same flask as used in Example 1, a mixture of 75 % by mole of $H(CF_2)_3CF=CHI$ (the vinyl compound) and 25 % by mole of $H(CF_2)_4CH_2I$ (the saturated compound) (8.72 g, 0.02 mole of the vinyl compound), 0.5 % palladium/carbon catalyst (0.3 g), potassium hydroxide (1.12 g, 0.02 mole) and water (5 ml) were charged. After purging the internal atmosphere with hydrogen, hydrogen gas kept at 101,3 kPa (1 atm) was introduced in the flask at room temperature (25°C) for 10 hours. The reaction system absorbed 510 cc of hydrogen gas. After the reaction, the organic phase was analyzed with gas chromatography (Porapak Type Q) to find that the saturated compound was not reacted and the conversion of the vinyl compound was 79.0 % by mole and the selectivity of $H(CF_2)_3CF=CH_2$ was 87.5 % by mole. The by-product was $H(CF_2)_3CHFCH_3$.

## Example 5

In a stainless steel reactor tube having an inner diameter of 19 mm and a length of 650 mm, 0.5 % palladium/ carbon catalyst (45 g) was filled. The length of the tube filled with the catalyst was 400 mm.

While heating the catalyst filled part at 150°C, a mixture of $H(CF_2)_3CF=CHCl$ and hydrogen (a molar ratio of 1:1) was introduced in the reactor tube at a contact time of about 15.0 seconds.

The reaction product was trapped in a cold trap cooled with a dry ice/methanol bath and washed with an aqueous solution of sodium hydroxide.

The organic phase was analyzed with gas chromatography (Porapak Type Q) to find that the conversion was 73.7 % by mole and the selectivity of $H(CF_2)_3CF=CH_2$ was 88.6 % by mole. The by-product was $H(CF_2)_3CHFCH_3$.

## Comparative Example

In a 200 ml autoclave equipped with a stirrer and a thermometer, $CClF=CF_2$ (2.33 g, 0.02 mole), 0.5 % palladium/carbon catalyst (0.3 g), potassium hydroxide (1.12 g, 0.02 mole) and water (5 ml) were charged and kept at room temperature (25°C). The internal pressure was 658,6 kPa (6.5 atm). Then, the autoclave was pressurized to 911,9 kPa (9 atm.) with hydrogen gas and the reaction was continued for 2 hours.

The internal gas in the autoclave was analyzed with gas chromatography (Porapak Type Q) to find that the conversion was 61.5 % by mole and the selectivity of $CHF=CF_2$ was 30.2 % by mole. The by-products were $CH_2FCHF_2$ and $CH_3-CHF_2$.

## Claims

1. A process for preparing a fluoroalkyl vinyl compound of the formula:
$$R_fCF = CH_2 \quad (I)$$
wherein $R_f$ is a fluoroalkyl group having 1 to 10 carbon atoms, which process comprises reacting a fluoroalkyl vinyl halide of the formula:

$$R_fCF = CHX \quad (II)$$

or

$$R_fCF = CX_2 \quad (III)$$

wherein $R_f$ is the same as defined above, and X is a chlorine atom, a bromine atom or an iodine atom with hydrogen in the presence of a palladium catalyst.

2. The process according to claim 1, wherein the number of the fluorine atoms in the fluoroalkyl group $R_f$ is larger than the number of the carbon atoms in the group.

3. The process according to claim 2, wherein the number of the fluorine atoms in the fluoroalkyl group $R_f$ is at least twice larger than the number of the carbon atoms in the group.

4. The process according to claim 1, wherein the fluoroalkyl group $R_f$ is selected from the group consisting of $CF_3-$, $CF_3C(CF_2)_3-$, $CF_3(CF_2)_5-$, $HCF_2-$, $H(CF_2)_3-$ and $(CF_3)_2CFCF_2-$.

5. The process according to claim 1, wherein the fluoroalkyl vinyl halide (II) is at least one selected from the group consisting of $H(CF_2)_3CF=CHCl$, $H(CF_2)_3CF=CHI$ and $CF_3CF=CHCl$.

6. The process according to claim 1, wherein the fluoroalkyl vinyl halide (III) is at least one selected from the group consisting of $H(CF_2)_3CF=CCl_2$, $H(CF_2)_3CF=CI_2$ and $CF_3CF=CCl_2$.

7. The process according to claim 1, wherein the amount of hydrogen is from 0.1 to 10 moles per one mole of the fluoroalkyl vinyl halide.

8. The process according to claim 7, wherein the amount of hydrogen is from 0.5 to 1.5 moles per one mole of the fluoroalkyl vinyl halide.

9. The process according to claim 1, wherein the palladium catalyst is supported on activated carbon.

10. The process according to claim 1, which is carried out in a solvent.

11. The process according to claim 10, wherein the solvent is water

12. The process according to claim 10, wherein the amount of the solvent is 0.3 to 20 times volume of the fluoroalkyl vinyl halide.

13. The process according to claim 1, wherein the reaction temperature is from 5 to 200°C.

14. The process according to claim 1, wherein the reaction pressure is from 50,7 kPa to 5066,2 kPa (0.5 to 50 atm.)

**Patentansprüche**

1. Verfahren zum Herstellen einer Fluoralkylvinylverbindung der Formel:
$$R_fCF = CH_2 \quad (I)$$
wobei $R_f$ eine Fluoralkylgruppe mit 1 bis 10 Kohlenstoffatomen ist, umfassend Umsetzen eines Fluoralkylvinylhalogenids der Formel
$$R_fCF = CHX \quad (II)$$
oder
$$R_fCF = CX_2 \quad (III),$$
wobei $R_f$ wie zuvor angegeben definiert ist, und X ein Chloratom, ein Bromatom oder ein Jodatom ist, mit Wasserstoff in Anwesenheit eines Palladiumkatalysators.

2. Verfahren nach Anspruch 1, wobei die Anzahl der Fluoratome in der Fluoralkylgruppe $R_f$ größer als die Anzahl der Kohlenstoffatome in der Gruppe ist.

3. Verfahren nach Anspruch 2, wobei die Anzahl der Fluoratome in der Fluoralkylgruppe $R_f$ mindestens zweimal größer als die Anzahl der Kohlenstoffatome in der Gruppe ist.

4. Verfahren nach Anspruch 1, wobei die Fluoralkylgruppe $R_f$ aus der Gruppe aus $CF_3-$, $CF_3C(CF_2)_3-$, $CF_3(CF_2)_5-$, $HCF_2-$, $H(CF_2)_3-$ und $(CF_3)_2CFCF_2-$ ausgewählt wird.

5. Verfahren nach Anspruch 1, wobei das Fluoralkylvinylhalogenid (II) mindestens eines aus der Gruppe aus $H(CF_2)_3CF=CHCl$, $H(CF_2)_3CF=CHI$ und $CF_3CF=CHCl$ ist.

6. Verfahren nach Anspruch 1, wobei das Fluoralkylvinylhalogenid (III) mindestens eines aus der Gruppe aus $H(CF_2)_3CF=CCl_2$, $H(CF_2)_3CF=CI_2$ und $CF_3CF=CCl_2$ ist.

7. Verfahren nach Anspruch 1, wobei die Wasserstoffmenge 0,1 bis 10 Mole pro 1 Mol des Fluoralkylvinylhalogenids ist.

8. Verfahren nach Anspruch 7, wobei die Wasserstoffmenge 0,5 bis 1,5 Mole pro 1 Mol des Fluoralkylvinylhalogenids ist.

9. Verfahren nach Anspruch 1, wobei der Palladiumkatalysator auf Aktivkohle aufgelagert ist.

10. Verfahren nach Anspruch 1, welches in einem Lösungsmittel durchgeführt wird.

11. Verfahren nach Anspruch 10, wobei das Lösungsmittel Wasser ist.

12. Verfahren nach Anspruch 10, wobei die Menge des Lösungsmittels das 0,3- bis 20-fache des Volumens des Fluoralkylvinylhalogenids ist.

13. Verfahren nach Anspruch 1, wobei die Reaktionstemperatur 5 bis 200°C beträgt.

14. Verfahren nach Anspruch 1, wobei der Reaktionsdruck 50,7 kPa bis 5066,2 kPa (0,5 bis 50 atm) beträgt.


**Revendications**

1. Procédé de préparation d'un composé fluoroalkylvinylique de formule :

$$R_fCF = CH_2 \quad (I)$$

dans lequel $R_f$ est un groupe fluoroalkyle contenant de 1 à 10 atomes de carbone, lequel procédé comprend la réaction d'un halogénure de fluoroalkylvinyle de formule :

$$R_fCF = CHX \quad (II)$$

ou

$$R_fCF = CX_2 \quad (III)$$

dans lequel $R_f$ est le même que celui défini ci-dessus, et X est un atome de chlore, un atome de brome ou un atome d'iode,
avec de l'hydrogène en présence d'un catalyseur au palladium.

2. Procédé selon la revendication 1, dans lequel le nombre d'atomes de fluor dans le groupement fluoroalkyle $R_f$ est supérieur au nombre d'atomes de carbone du groupement.

3. Procédé selon la revendication 2, dans lequel le nombre d'atomes de fluor dans le groupement fluoroalkyle $R_f$ est au moins deux fois plus important que le nombre d'atomes de carbone du groupement.

4. Procédé selon la revendication 1, dans lequel le groupement fluoroalkyle $R_f$ est choisi parmi les suivants : $CF_3-$, $CF_3C(CF_2)_3-$, $CF_3(CF_2)_5-$, $HCF_2-$, $H(CF_2)_3-$ et $(CF_3)_2CFCF_2-$.

5. Procédé selon la revendication 1, dans lequel l'halogénure fluoroalkylvinyle (II) est au moins l'un des suivants : $H(CF_2)_3CF=CHCl$, $H(CF_2)_3CF=CHI$ et $CF_3CF=CHCl$.

6. Procédé selon la revendication 1, dans lequel l'halogénure de fluoroalkylvinyle (III) est au moins l'un des suivants : $H(CF_2)_3CF=CCl_2$, $H(CF_2)_3CF=Cl_2$ et $CF_3CF=CCl_2$.

7. Procédé selon la revendication 1, dans lequel la quantité d'hydrogène est de 0,1 à 10 mol par mole de l'halogénure de fluoroalkylvinyle.

8. Procédé selon la revendication 7, dans lequel la quantité d'hydrogène est de 0,5 à 1,5 mol par mole de l'halogénure de fluoroalkylvinyle.

9. Procédé selon la revendication 1, dans lequel le catalyseur au palladium est supporté sur charbon actif.

10. Procédé selon la revendication 1, qui est mis en oeuvre dans un solvant.

11. Procédé selon la revendication 10, dans lequel le solvant est l'eau.

12. Procédé selon la revendication 10, dans lequel la quantité de solvant est de 0,3 à 20 fois le volume de l'halogénure de fluoroalkylvinyle.

13. Procédé selon la revendication 1, dans lequel la température de réaction est comprise entre 5 et 200°C.

14. Procédé selon la revendication 1, dans lequel la pression de réaction est comprise entre 50,7 kPa et 5066,2 kPa (0,5 et 50 atm).